(19) Europäisches Patentamt
European Patent Office
Office européen des brevets

(11) Veröffentlichungsnummer: **0 081 141**
**B1**

(12) **EUROPÄISCHE PATENTSCHRIFT**

(45) Veröffentlichungstag der Patentschrift: **31.07.85**

(21) Anmeldenummer: **82110858.6**

(22) Anmeldetag: **24.11.82**

(51) Int. Cl.[4]: **C 07 D 261/08**, C 07 D 261/18, C 07 D 271/06, C 07 D 271/10, C 07 D 231/12, C 07 D 233/68, C 07 D 277/24, C 07 D 277/64, C 07 D 285/08, C 07 D 285/12, A 01 N 47/30

(54) **Harnstoffderivate, Verfahren zu ihrer Herstellung und ihre Verwendung zur Bekämpfung unerwünschten Pflanzenwuchses.**

(30) Priorität: **05.12.81 DE 3148291**

(43) Veröffentlichungstag der Anmeldung: **15.06.83 Patentblatt 83/24**

(45) Bekanntmachung des Hinweises auf die Patenterteilung: **31.07.85 Patentblatt 85/31**

(84) Benannte Vertragsstaaten: **AT BE CH DE FR GB IT LI NL**

(56) Entgegenhaltungen:
**EP - A - 0 022 750**
**DE - A - 2 101 938**
**DE - A - 2 801 509**
**DE - A - 2 950 019**
**FR - A - 2 128 505**
**US - A - 4 003 906**
**US - A - 4 028 092**
**US - A - 4 289 903**

**CHEMICAL ABSTRACTS, Band 96, Nr. 9, 1. März 1982, Seite 594, Nr. 68809h, Columbus, Ohio, USA**
**CHEMICAL ABSTRACTS, Band 97, Nr. 17, 25. Oktober 1982, Seite 689, Nr. 144858m, Columbus, Ohio, USA**
**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Becker, Rainer, Dr., Im Haseneck 22, D-6702 Bad Duerkheim (DE)**
Erfinder: **Theobald, Hans, Dr., Parkstrasse 2, D-6703 Limburgerhof (DE)**
Erfinder: **Schirmer, Ulrich, Dr., Berghalde 79, D-6900 Heidelberg (DE)**
Erfinder: **Spiegler, Wolfgang, Dr., Amsterdamer Strasse 16, D-6700 Ludwigshafen (DE)**
Erfinder: **Seufert, Walter, Dr., Bruesseler Ring 28, D-6700 Ludwigshafen (DE)**
Erfinder: **Wuerzer, Bruno, Dr., Ruedigerstrasse 13, D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft Harnstoffderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Es ist bekannt, daß N-Phenyl-harnstoffe, die am Phenylring noch einen durch einen Heterocyclus substituierten Alkoxyrest tragen, herbizid wirksam sind, so z.B. N-Thienylalkyloxyphenyl harnstoffe (JP-A-81/133 282), N-Dioxolanylalkyloxyphenyl-harnstoffe (US A 4 289 903), N-Pyridylalkyloxyphenyl-harnstoffe (US-A-4 028 092; US-A-4 003 906) und N-Benzimidazolylmethoxyphenyl-harnstoffe (JP-A-82/106 667).

Es wurde gefunden, daß Harnstoffderivate der Formel

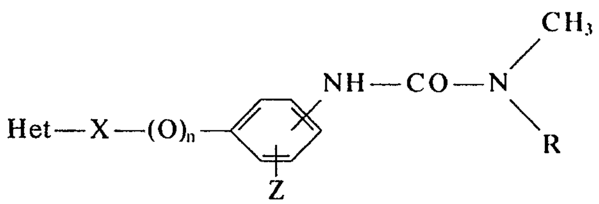

(I)

in der

R Wasserstoff, einen Alkyl- oder Alkoxyrest mit bis zu 4 Kohlenstoffatomen, Allyl oder 1-Methyl-prop-2-inyl,
X einen unverzweigten oder verzweigten Alkylenrest mit bis zu 4 Kohlenstoffatomen,
Z Wasserstoff, Halogen, Methyl, Trifluormethyl,
Het einen gegebenenfalls durch Halogen, Alkyl, Halogenalkyl, Alkoxy, Halogenalkoxy, Alkoxyalkyl oder Alkoxycarbonyl mit jeweils bis zu 5 Kohlenstoffatomen oder durch gegebenenfalls substituiertes Phenyl oder durch gegebenenfalls substituiertes Benzyl substituierten Isoxazol-, Oxazol-, Thiazol-, Oxadiazol-, Thiadiazol-, Pyrazol- oder Triazolring oder einen entsprechenden benzoanellierten, gegebenenfalls in gleicher Weise substituierten Fünfringheterocyclus und
n 0 oder 1 bedeuten,

herbizide Wirkung haben und für Kulturpflanzen selektiv sind.

In Formel I bedeutet R Wasserstoff, einen Alkyl- oder Alkoxyrest mit bis zu 4 Kohlenstoffatomen, wie Methyl, Methoxy, Ethoxy, sowie Allyl oder 1-Methyl-prop-2-inyl. R bedeutet vorzugsweise Methyl oder Methoxy, insbesondere Methoxy.

X bedeutet einen unverzweigten oder verzweigten Alkylenrest mit bis zu 4 Kohlenstoffatomen, wie Methylen, Dimethylen, 1-Methyl-dimethylen, Trimethylen.

Z bedeutet außer Wasserstoff, Methyl und Trifluormethyl, Halogen, wie Chlor, Fluor, Brom, insbesondere Chlor.

Het steht für Isoxazol-, Oxazol, Thiazol-, Oxadiazol-, Thiadiazol-, Pyrazol- oder Triazolringe oder entsprechende Fünfringheterocyclen, beispielsweise Benzoxazol- oder Benzothiazolringe, die durch Halogen, wie Chlor, Fluor, Brom, Alkyl mit bis zu 5 Kohlenstoffatomen, wie Methyl, Ethyl, Isopropyl, tert.-Butyl, Halogenalkyl mit bis zu 5 Kohlenstoffatomen, wie Trifluormethyl, Alkoxy mit bis zu 5 Kohlenstoffatomen, wie Methoxy, Ethoxy, Halogenalkoxy mit bis zu 5 Kohlenstoffatomen, wie Difluormethoxy, Trifluormethoxy, Alkoxyalkyl mit bis zu 5 Kohlenstoffatomen, wie Methoxymethyl, Ethoxymethyl, durch Alkoxycarbonyl mit bis zu 5 Kohlenstoffatomen, wie Methoxycarbonyl, Ethoxycarbonyl oder durch gegebenenfalls durch Halogen, wie Chlor, Alkyl, wie Methyl, Alkoxy, wie Methoxy, oder Nitro substituiertes Phenyl oder Benzyl substituiert sein können.

Bevorzugte Verbindungen der Formel I sind solche, bei denen Het für einen gegebenenfalls durch Halogen, Alkyl, Halogenalkyl, Alkoxy, Halogenalkoxy, Alkoxyalkyl oder Alkoxycarbonyl mit jeweils bis zu 5 Kohlenstoffatomen oder durch gegebenenfalls substituiertes Phenyl oder durch gegebenenfalls substituiertes Benzyl substituierten Benzthiazolylrest steht.

Man erhält die Verbindungen der Formel I, bei denen n 1 bedeutet, durch Umsetzung von Verbindungen der Formel

$$Het-X-Hal \qquad (II)$$

in der Het und X die oben genannten Bedeutungen haben und Hal für Halogen, insbesondere für Chlor, steht, mit einem Phenol der Formel

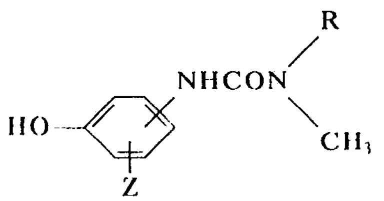

(III)

in der R und Z die oben genannten Bedeutungen haben, in einem inerten Lösungsmittel bei einer Temperatur zwischen 0 und 150°C, vorzugsweise zwischen 50 und 100°C.

Die Umsetzung wird zweckmäßigerweise in Gegenwart einer Base durchgeführt, wobei die Menge an Base, bezogen auf die Verbindung der Formel II, 1,0 bis 1,5 Mol beträgt. Geeignete Basen sind Alkalicarbonate, Alkalihydrogencarbonate, Alkalihydroxide, wie Natriumcarbonat, Natriumhydrogencarbonat, Kaliumcarbonat, Natriumhydroxid, Kaliumhydroxid.

Als Lösungsmittel kommen polare organische Lösungsmittel, wie Aceton, Acetonitril, Dimethylformamid, Dimethylsulfoxid, oder Wasser in Betracht. Wird die Reaktion in wäßrigem Medium durchgeführt, wird zweckmäßigerweise ein Phasentransferkatalysator, z. B. Tetrabutylammoniumiodid, zugesetzt. Die Menge an Phasentransferkatalysator beträgt etwa 0,01 bis 0,1 Mol, bezogen auf Verbindung der Formel II.

Die Reaktionskomponenten werden in äquimolarem Verhältnis umgesetzt. Ein Überschuß der einen oder anderen Komponente stört nicht.

Man erhält die Verbindungen der Formel I, bei denen n 1 bedeutet, auch durch Umsetzung von Verbindungen der Formel

Het—X—OH (IV)

in der Het und X die oben genannten Bedeutungen haben, mit Halogennitroaromaten der Formel

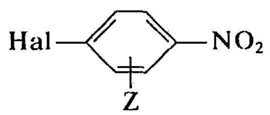

(V)

in der Z die oben genannten Bedeutungen hat und Hal für Halogen steht, Reduktion der Nitroverbindung und Umsetzung des Anilinderivats mit

a) einem Isocyanat der Formel RNCO oder
b) einem Carbaminsäurechlorid der Formel

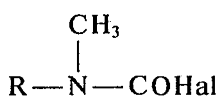

wobei R jeweils die oben genannten Bedeutungen hat und Hal für Halogen steht. Das Anilinderivat kann auch phosgeniert und mit einem Amin der Formel $HN(CH_3)R$ zum Harnstoffderivat umgesetzt werden.

Die Umsetzung der Verbindungen der Formel IV mit den Verbindungen der Formel V wird unter den gleichen Bedingungen durchgeführt wie die Umsetzung der Verbindungen der Formel II mit den Verbindungen der Formel III.

Die Reduktion der erhaltenen Nitroverbindungen erfolgt in bekannter Weise durch katalytische Hydrierung mit Raney-Nickel oder Pd/C bzw. durch Umsetzung mit Reduktionsmitteln, wie Zinn(II)-chlorid oder Eisenpulver in Salzsäure.

Die Überführung der dabei erhaltenen Anilinderivate in die Harnstoffe erfolgt ebenfalls in bekannter Weise durch Umsetzung mit Methylisocyanat in inerten Lösungsmitteln, wie Aceton, Acetonitril, Toluol oder Tetrahydrofuran, bzw. durch Umsetzung mit Carbaminsäurechloriden in Pyridin zwischen 0 und 50°C oder in inerten Lösungsmitteln, wie Aceton, Tetrahydrofuran, Acetonitril, unter Zuhilfenahme einer Base, wie Triethylamin, Pyridin, einem Alkalicarbonat oder einem Alkalihydrogencarbonat.

Verbindungen der Formel I, in der n 1 bedeutet, erhält man auch durch Umsetzung von Verbindungen der Formel

Het—X—Hal (VI)

in der Het und X die oben genannten Bedeutungen haben und Hal für Halogen steht, mit Nitrophenolderivaten der Formel

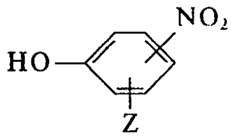

(VII)

in der Z die oben genannten Bedeutungen hat, in einem inerten Lösungsmittel bei einer Temperatur zwischen 0 und 150°C, vorzugsweise zwischen 50 und 100°C,
Reduktion der Nitroverbindung und Umsetzung des Anilinderivates mit einem Isocyanat der Formel

RNCO

oder einem Carbaminsäurechlorid der Formel

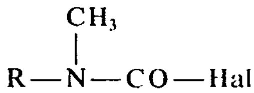

wobei R jeweils die oben genannten Bedeutungen hat und Hal für Halogen, vorzugsweise für Chlor, steht. Das Anilinderivat kann auch phosgeniert und mit einem Amin der Formel $HN(CH_3)R$ zum Harnstoffderivat umgesetzt werden.

Die Umsetzung der Verbindungen der Formel VI mit den Verbindungen der Formel VII wird unter den gleichen Bedingungen durchgeführt wie die Umsetzung der Verbindungen der Formel II mit den Verbindungen der Formel III.

Verbindungen der Formel I, in der n 0 bedeutet, erhält man durch Umsetzung von Aldehyden der Formel

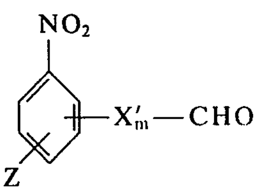

(VIII)

in der X' einen unverzweigten oder verzweigten Alkylenrest mit 1 oder 2 Kohlenstoffatomen und m 0 oder 1 bedeuten und Z die oben genannten Bedeutungen hat,
mit einer aktiven Methylenverbindung der Formel

$$Het-CH_3 \qquad (IX)$$

in der Het die oben genannten Bedeutungen hat, anschließende Hydrierung des Reaktionsproduktes und Umsetzung mit einem Carbaminsäurehalogenid der Formel

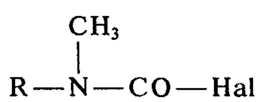

oder mit einem Isocyanat der Formel

RNCO

wobei R jeweils die oben genannten Bedeutungen hat und Hal für Halogen, vorzugsweise für Chlor, steht.

Die Umsetzung der Aldehyde der Formel VIII mit den Methylenverbindungen der Formel IX werden in einem inerten Lösungsmittel, beispielsweise in Essigsäure unter Zugabe von Acetanhydrid, bei Temperaturen zwischen 50 und 150°C durchgeführt. Die Reaktionskomponenten werden in äquimolarem Verhältnis eingesetzt; ein Überschuß der einen oder anderen Komponente stört nicht.

Die Reduktion der so erhaltenen Nitroverbindungen erfolgt in bekannter Weise durch katalytische Hydrierung unter Zugabe von z. B. Raney-Nickel oder Pd/C; die C—C-Doppelbindung wird im Zuge dieser Reaktion ebenfalls hydriert.

Die Überführung der dabei erhaltenen Anilinderivate in die Harnstoffe erfolgt in ebenfalls bekannter Weise durch Umsetzung mit Methylenisocyanat in einem inerten Lösungsmittel, wie Toluol, Aceton, Acetonitril, oder durch Umsetzung mit Carbaminsäurechloriden in Pyridin bei einer Temperatur zwischen 0 und 5°C oder in inerten Lösungsmitteln, wie Aceton, Tetrahydrofuran, Acetonitril, unter Zuhilfenahme einer organischen Base, wie Triethylamin oder Pyridin, oder einer anorganischen Base, wie

Alkalicarbonat, Alkalihydrogencarbonat.

Verbindungen der Formel I, in der n 0 und Het einen gegebenenfalls substituierten Pyrazol- oder Isoxazolring bedeuten, lassen sich aus Aldehyden der Formel

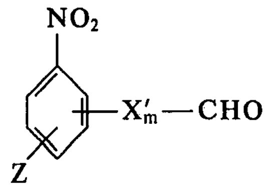

(VIII)

in der X' eine unverzweigte oder verzweigte Alkylengruppe mit 1 oder 2 Kohlenstoffatomen und m 0 oder 1 bedeuten und Z die oben genannten Bedeutungen hat,
mit einer aktivierten Methylenverbindung der Formel

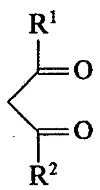

(X)

in der
$R^1$ und $R^2$ jeweils Alkyl mit bis zu 5 Kohlenstoffatomen bedeuten,
durch anschließende Hydrierung und Umsetzung mit einem Hydrazinderivat der Formel $NH_2NHR^3$, in der $R^3$ Wasserstoff, Alkyl oder Phenyl bedeutet, oder mit Hydroxylamin herstellen.

Die Umsetzung der Verbindungen der Formel VIII mit Verbindungen der Formel X wird in einem inerten Lösungsmittel, beispielsweise in Toluol, unter Zugabe katalytischer Mengen von z. B. Eisessig/Piperidin bei einer Temperatur zwischen 50 und 150°C unter Wasserauskreisung durchgeführt. Die dabei als Zwischenprodukt gewonnene ungesättigte Verbindung

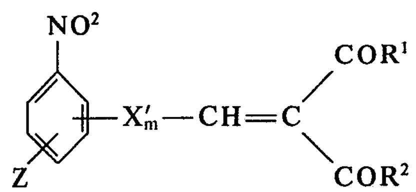

wird in bekannter Weise mit Raney-Nickel oder Pd/C hydriert und dann mit gegebenenfalls substituierten Hydrazinen der Formel $NH_2NHR^3$, in der $R^3$ Wasserstoff, Alkyl oder Phenyl bedeutet, oder mit Hydroxylamin cyclisiert. Die so erhaltenen Aniline lassen sich nach den oben beschriebenen Methoden in die Harnstoffe der Formel I überführen.

Die folgenden Beispiele erläutern die Durchführung der erfindungsgemäßen Verfahren. Gewichtsteile verhalten sich zu Volumenteilen wie Kilogramm zu Liter.

Beispiel 1

113 Gewichtsteile 3-Methyl-5-hydroxymethyl-isoxazol und 141 Gewichtsteile p-Fluornitrobenzol werden in 200 Volumenteilen Dimethylformamid mit 138 Gewichtsteilen Kaliumcarbonat versetzt und 20 Stunden bei 135°C gerührt. Nach dem Abkühlen wird in Eiswasser eingerührt, und das 4-(3-Methyl-isoxazol-5-yl-methoxy)-nitrobenzol wird abgesaugt; Ausbeute 201 g; Schmelzpunkt 127–128°C.

$C_{11}H_{10}N_2O_4/M = 234$

| | | | |
|---|---|---|---|
| Ber.: | C 56,41 % | H 4,30 % | N 11,96 % |
| Gef.: | C 56,7 % | H 4,3 % | N 11,9 % |

220 Gewichtsteile 4-(3-Methyl-isoxazol-5-yl-methoxy)-nitrobenzol werden in 365 Gewichtsteilen Ethanol und 446 Gewichtsteilen conc. Salzsäure suspendiert und portionsweise mit 767 g $SnCl_2 \cdot 2\,H_2O$ versetzt. Die Temperatur wird dabei zwischen 50 und 60°C gehalten. Es wird noch 2 Stunden lang bei 60°C nachgerührt, die erhaltene Lösung dann in 2480 Gewichtsteile Eis und 1622 Gewichtsteile 50%ige Natronlauge eingerührt. Das 4-(3-Methyl-isoxazol-5-yl-methoxy)-anilin wird dann abgesaugt; Ausbeute 139 g; Schmelzpunkt 67–69°C.

$C_{11}H_{12}N_2O_2/M = 204$
Ber.: C 64,69 % H 5,92 % N 13,72 %
Gef.: C 64,1 % H 5,8 % N 13,6 %

20,4 Gewichtsteile 4-(3-Methyl-isoxazol-5-yl)methoxyanilin werden in 150 Volumenteilen Pyridin gelöst und bei 20°C tropfenweise mit 10,8 Gewichtsteilen Dimethylcarbaminsäurechlorid versetzt. Nach 20 Stunden wird in Eiswasser/Salzsäure eingerührt, und der N-[4-(3-Methyl-isoxazol-5-yl-methoxy)-phenyl]-N',N'-dimethylharnstoff wird abgesaugt; Ausbeute 14,1 g; Schmelzpunkt 121—122°C.

$C_{14}H_{17}N_3O_3/M = 275$
Ber.: C 61,08 % H 6,22 % N 15,26 %
Gef.: C 61,5 % H 5,9 % N 14,6 %

## Beispiel 2

13,3 Gewichtsteile 2-Methyl-5-chlormethyl-1,3,4-oxadiazol werden in 200 Volumenteilen Aceton mit 18 Gewichtsteilen N-(3-Hydroxyphenyl)-N',N'-dimethylharnstoff und 13,8g Kaliumcarbonat 7 Stunden unter Rückfluß erhitzt.
Nach dem Abkühlen wird in Eiswasser eingerührt und abgesaugt. Man erhält 15,6 g N-[3-(2-Methyl-1,3,4-oxadiazol-5-yl-methoxy)-phenyl]-N',N'-dimethylharnstoff vom Schmelzpunkt 102—105°C.

$C_{13}H_{16}N_4O_3/M = 276$
Ber.: C 56,51 % H 5,84 % N 20,28 %
Gef.: C 56,1 % H 5,8 % N 19,5 %

## Beispiel 3

149 Gewichtsteile 2-Methylbenzthiazol und 151 Gewichtsteile 4-Nitrobenzaldehyd werden in einem Gemisch aus 400 Volumenteilen Eisessig und 400 Volumenteilen Acetanhydrid gelöst und 5 Stunden bei 130°C gerührt. Anschließend wird auf 70°C gekühlt, und 800 ml Ethanol werden vorsichtig zugefügt. Das erhaltene Gemisch wird gekühlt und abgesaugt.

Ausbeute: 168 g β-(Benzthiazolyl-2)-4-nitrostyrol vom Schmelzpunkt 235—237°C.

$C_{15}H_{10}N_2SO_2/M = 282$
Ber.: C 63,82 % H 3,57 % N 9,92 % S 11,36 %
Gef.: C 64,1 % H 3,6 % N 9,9 % S 11,2 %

150 Gewichtsteile β-(2-Benzthiazolyl)-4-nitrostyrol werden in 1500 Volumenteilen Tetrahydrofuran gelöst und mit 15 Gewichtsteilen Pd/C (10%) versetzt. Nach Spülen mit Stickstoff wird Wasserstoff eingeleitet. Nach einigen Stunden ist die Wasserstoffaufnahme (44 l) beendet, es wird wiederum mit Stickstoff gespült, dann wird vom Katalysator abfiltriert und eingeengt. Man erhält 135,6 g 4-(β-Benzthiazol-2-yl-ethyl)-anilin vom Schmelzpunkt 79—81°C.
25,4 Gewichtsteile 4-(β-Benzthiazol-2-yl-ethyl)-anilin werden in 150 Volumenteilen Tetrahydrofuran gelöst und mit 13,8 Gewichtsteilen Kaliumcarbonat versetzt. Anschließend werden 13,6 Gewichtsteile N-Methyl-N-methoxycarbaminsäurechlorid zugetropft. Nach mehrstündigem Rühren wird in Eiswasser gegeben, mit Methylenchlorid extrahiert und eingeengt. Der erhaltene Feststoff wird aus Cyclohexan umkristallisiert.

Ausbeute: 23,3 g N-[4-(β-Benzthiazol-2-yl)-ethyl-phenyl]-N'-methyl-N'-methoxyharnstoff vom Schmelzpunkt 85—87°C.

$C_{18}H_{19}N_3O_2S/M = 341$
Ber.: C 63,32 % H 5,61 % N 12,31 % S 9,39 %
Gef.: C 63,3 % H 5,5 % N 12,1 % S 9,8 %

Die folgenden Verbindungen der Formel I lassen sich nach den oben beschriebenen Methoden herstellen:

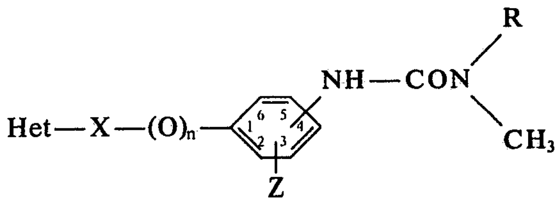

| Verbindung Nr. | Het | X | n | Z | Stellung der Harnstoffgruppe | R | Fp [°C] |
|---|---|---|---|---|---|---|---|
| 1 | Benzthiazolyl-2 | $-CH_2-CH_2-$ | 0 | H | 3 | $CH_3$ | 137–139 |
| 2 | 5-Phenyl-1,2,4-oxadiazolyl-3 | $-CH_2-$ | 1 | H | 3 | $OCH_3$ | |
| 3 | 5-Methyl-1,3,4-thiadiazolyl-2 | $-CH_2-$ | 1 | H | 4 | $OCH_3$ | 139–145 |
| 4 | 3-tert.-Butylisoxazolyl-5 | $-CH_2-CH_2-$ | 1 | H | 4 | $OCH_3$ | |
| 5 | 3-Methylisoxazolyl-5 | $-CH(CH_3)-$ | 1 | H | 4 | $CH_3$ | zähes Öl |
| 6 | 5-Phenyl-1,2,4-oxadiazolyl-3 | $-CH_2-$ | 1 | H | 4 | $OCH_3$ | |
| 7 | 2-Phenylthiazolyl-4 | $-CH_2-$ | 1 | H | 4 | $OCH_3$ | 94–96 |
| 8 | 1-Phenyl-3,5-dimethyl-pyrazolyl-4 | $-CH_2-$ | 0 | H | 4 | $OCH_3$ | |
| 9 | Benzthiazolyl-2 | $-CH_2-CH_2-$ | 0 | H | 3 | $OCH_3$ | 95–98 |
| 10 | 1,3,5-Trimethyl-pyrazolyl-4 | $-CH_2-$ | 0 | H | 4 | $OCH_3$ | |
| 11 | 5-Methyl-1,3,4-oxadiazolyl-2 | $-CH_2-$ | 1 | H | 3 | $CH_3$ | 102–105 |
| 12 | 3-Methyl-1,2,4-thiadiazolyl-5 | $-CH_2-$ | 1 | H | 4 | $OCH_3$ | |
| 13 | 3-Methyl-isoxazolyl-5 | $-CH(CH_3)-$ | 1 | H | 4 | $OCH_3$ | zähes Öl |
| 14 | 1,3,5-Trimethylpyrazolyl-4 | $-CH_2CH_2-$ | 0 | H | 4 | $OCH_3$ | |
| 15 | 3-tert.-Butyl-isoxazolyl-5 | $-CH_2-$ | 1 | H | 4 | $CH_3$ | 125–128 |
| 16 | 5-Phenyl-1,2,4-oxadiazolyl-3 | $-CH_2-$ | 1 | H | 4 | $CH_3$ | |
| 17 | 3-Phenyl-isoxazolyl-5 | $-CH_2-$ | 1 | H | 4 | $OCH_3$ | 123–125 |
| 18 | 3-Methylisoxazolyl-5 | $-CH_2CH_2-$ | 1 | H | 4 | $OCH_3$ | |

Fortsetzung

| Verbindung Nr. | Het | X | n | Z | Stellung der Harnstoffgruppe | R | Fp [°C] |
|---|---|---|---|---|---|---|---|
| 19 | 3-Methylisoxazolyl-5 | $-CH_2-$ | 1 | 2-Cl | 4 | $CH_3$ | 130–131 |
| 20 | 3-tert.-Butyl-isoxazolyl-5 | $-CH_2CH_2CH_2-$ | 0 | H | 4 | $OCH_3$ | |
| 21 | 3-Methylisoxazolyl-5 | $-CH_2-$ | 1 | H | 3 | $CH_3$ | 147–148 |
| 22 | 5-tert.-Butyl-1,2,4-oxadiazolyl-3 | $-CH_2-$ | 1 | H | 4 | $OCH_3$ | |
| 23 | 3-Ethylisoxazolyl-5 | $-CH_2-$ | 1 | H | 4 | $CH_3$ | 119–120 |
| 24 | 3-tert.-Butyl-isoxazolyl-5 | $-CH_2CH_2-$ | 1 | H | 4 | $CH_3$ | |
| 25 | 5-Methyl-1,3,4-thiadiazolyl-2 | $-CH_2-$ | 1 | H | 4 | $CH_3$ | 168–171 |
| 26 | Benzthiazolyl-2 | $-CH_2CH_2-$ | 0 | H | 4 | $CH_3$ | 170–172 |
| 28 | 5-Methyl-1,3,4-thiadiazolyl-2 | $-CH_2-$ | 1 | H | 3 | $CH_3$ | 155–157 |
| 29 | 1-Phenyl-3,5-dimethylpyrazolyl-4 | $-CH_2-$ | 0 | H | 3 | $OCH_3$ | Öl |
| 30 | Isoxazolyl-5 | $-CH_2-$ | 1 | H | 4 | $OCH_3$ | 109–111 |
| 31 | 5-Methyl-1,2,4-oxadiazolyl-3 | $-CH_2-$ | 1 | H | 4 | $CH_3$ | |
| 32 | 3-Methylisoxazolyl-5 | $-CH_2-$ | 1 | H | 4 | H | 176–180 |
| 33 | 3-Methylisoxazolyl-5 | $-CH_2CH_2-$ | 1 | H | 4 | $CH_3$ | |
| 34 | 3-Ethoxycarbonylisoxazolyl-5 | $-CH_2-$ | 1 | H | 4 | $OCH_3$ | 72–75 |
| 35 | Benzthiazolyl-2 | $-CH_2-$ | 1 | H | 4 | $OCH_3$ | |
| 36 | 3-Methylisoxazolyl-5 | $-CH_2-$ | 1 | H | 3 | $OCH_3$ | 91–92 |

Fortsetzung

| Verbindung Nr. | Het | X | n | Z | Stellung der Harnstoffgruppe | R | Fp [°C] |
|---|---|---|---|---|---|---|---|
| 37 | 5-Methyl-1,2,4-oxadiazolyl-3 | $-CH_2-$ | 1 | H | 4 | $OCH_3$ | |
| 38 | 5-Methyl-1,3,4-oxadiazolyl-2 | $-CH_2-$ | 1 | H | 4 | $OCH_3$ | 90–93 |
| 40 | 3-(4-Chlorphenyl)-isoxazolyl-5 | $-CH_2-$ | 1 | H | 4 | $CH_3$ | 201–203 |
| 42 | 3-Methoxymethylisoxazolyl-5 | $-CH_2-$ | 1 | H | 4 | H | 100–101 |
| 43 | 3-Methylisoxazolyl-5 | $-CH_2-$ | 1 | 2-$CF_3$ | 4 | $CH_3$ | |
| 44 | 3-Phenylisoxazolyl-5 | $-CH_2-$ | 1 | H | 4 | $CH_3$ | 165–167 |
| 45 | 1,3,5-Trimethylpyrazolyl-4 | $-CH_2CH_2CH_2-$ | 0 | H | 4 | $OCH_3$ | |
| 46 | 3-Ethylisoxazolyl-5 | $-CH_2-$ | 1 | H | 4 | $OCH_3$ | 72–73 |
| 47 | 1-Phenyl-3,5-dimethylpyrazolyl-4 | $-CH_2-$ | 0 | H | 4 | $CH_3$ | |
| 48 | 2-Methylthiazolyl-4 | $-CH_2-$ | 1 | H | 4 | $OCH_3$ | 65–67 |
| 49 | Benzthiazolyl-2 | $-CH_2CH_2CH_2-$ | 0 | H | 4 | $OCH_3$ | |
| 50 | 3-Methylisoxazolyl-5 | $-CH_2-$ | 1 | H | 4 | $OCH_3$ | 109–112 |
| 51 | 1,3,5-Trimethylpyrazolyl-4 | $-CH_2CH_2CH_2-$ | 0 | H | 4 | $CH_3$ | |
| 52 | 3-Methylisoxazolyl-5 | $-CH_2-$ | 1 | 2-Cl | 4 | $OCH_3$ | 141–142 |
| 53 | 3,5-Dimethylisoxazolyl-4 | $-CH_2CH_2CH_2-$ | 0 | H | 4 | $OCH_3$ | |
| 54 | 1,3,5-Trimethylpyrazolyl-4 | $-CH_2-$ | 0 | H | 3 | $OCH_3$ | 108–110 |
| 55 | 3-Methylisoxazolyl-5 | $-CH_2-$ | 1 | 2-$CF_3$ | 4 | $OCH_3$ | 105–107 |

Fortsetzung

| Verbindung Nr. | Het | X | n | Z | Stellung der Harnstoffgruppe | R | Fp [°C] |
|---|---|---|---|---|---|---|---|
| 56 | 3-Methylisoxazolyl-5 | $-CH_2-$ | 1 | H | 4 | $CH_3$ | 121–122 |
| 57 | 3,5-Dimethylisoxazolyl-4 | $-CH_2-$ | 0 | H | 4 | $CH_3$ | |
| 58 | Isoxazolyl-5 | $-CH_2-$ | 1 | H | 4 | H | 135–136 |
| 59 | 3,5-Dimethylisoxazolyl-4 | $-CH_2CH_2-$ | 0 | H | 4 | $OCH_3$ | |
| 60 | 3-Ethyloxadiazolyl-5 | $-CH_2-$ | 1 | H | 4 | H | 144–146 |
| 62 | 3-(4-Chlorphenyl)-isoxazolyl-5 | $-CH_2-$ | 1 | H | 4 | $OCH_3$ | 152–154 |
| 64 | 3-tert.-Butylisoxazolyl-5 | $-CH_2-$ | 1 | H | 4 | $OCH_3$ | 84–85 |
| 65 | 3-tert.-Butylisoxazolyl-5 | $-CH_2CH_2CH_2-$ | 0 | H | 3 | $OCH_3$ | |
| 66 | 3,5-Dimethylisoxazolyl-4 | $-CH_2-$ | 1 | H | 3 | $OCH_3$ | 112–114 |
| 67 | 1,2,4-Triazol-1-yl | $-CH_2CH(CH_3)-$ | 1 | H | 4 | $OCH_3$ | |
| 68 | 3-Isopropylisoxazolyl-5 | $-CH_2-$ | 1 | H | 4 | H | 111–113 |
| 69 | Benzthiazolyl-2 | $-CH_2CH_2-$ | 1 | H | 4 | $OCH_3$ | |
| 70 | Isoxazolyl-5 | $-CH_2-$ | 1 | H | 4 | $CH_3$ | 112–113 |
| 71 | 3,5-Dimethylisoxazolyl-4 | $-CH_2-$ | 0 | H | 4 | $OCH_3$ | |
| 72 | 2-Methylthiazolyl-4 | $-CH_2-$ | 1 | H | 3 | $OCH_3$ | 115–117 |
| 73 | 3-tert.-Butylisoxazolyl-5 | $-CH_2CH_2CH_2-$ | 0 | H | 4 | $CH_3$ | |
| 74 | 3-Methylisoxazolyl-5 | $-CH_2-$ | 1 | 2-$CH_3$ | 4 | $OCH_3$ | |

Fortsetzung

| Verbindung Nr. | Het | X | n | Z | Stellung der Harnstoffgruppe | R | Fp [°C] |
|---|---|---|---|---|---|---|---|
| 75 | Benzthiazolyl-2 | $-CH_2CH_2-$ | 0 | H | 4 | $OCH_3$ | 85–87 |
| 76 | 3,5-Dimethylisoxazolyl-4 | $-CH_2CH_2CH_2-$ | 0 | H | 3 | $OCH_3$ | |
| 77 | 3,5-Dimethylisoxazolyl-4 | $-CH_2CH_2CH_2-$ | 0 | H | 3 | $CH_3$ | |
| 78 | 1,3,5-Trimethylpyrazolyl-4 | $-CH_2CH_2CH_2-$ | 0 | H | 3 | $OCH_3$ | |
| 79 | 1,3,5-Trimethylpyrazolyl-4 | $-CH_2CH_2CH_2-$ | 0 | H | 3 | $CH_3$ | |
| 80 | 2-(4-Chlorbenzyl)-thiazolyl-4 | $-CH_2-$ | 1 | H | 3 | $OCH_3$ | 97–99 |
| 81 | 2-(4-Chlorbenzyl)-thiazolyl-4 | $-CH_2-$ | 1 | H | 3 | $CH_3$ | |
| 82 | 2-(4-Chlorbenzyl)-thiazolyl-4 | $-CH_2-$ | 1 | H | 4 | $OCH_3$ | 110–112 |
| 83 | 2-(4-Chlorbenzyl)-thiazolyl-4 | $-CH_2-$ | 1 | H | 4 | $CH_3$ | |
| 84 | 2-(4-Chlorbenzyl)-thiazolyl-4 | $-CH_2-$ | 1 | H | 3 | $OCH_3$ | 116–117 |
| 85 | 2-(4-Chlorphenyl)-thiazolyl-4 | $-CH_2-$ | 1 | H | 3 | $CH_3$ | |
| 86 | 2-(4-Chlorphenyl)-thiazolyl-4 | $-CH_2-$ | 1 | H | 4 | $OCH_3$ | 147–149 |
| 87 | 2-(4-Chlorphenyl)-thiazolyl-4 | $-CH_2-$ | 1 | H | 4 | $CH_3$ | |
| 88 | 2-(4-Methoxyphenyl)-thiazolyl-4 | $-CH_2-$ | 1 | H | 3 | $OCH_3$ | 111–114 |
| 89 | 2-(4-Methoxyphenyl)thiazolyl-4 | $-CH_2-$ | 1 | H | 3 | $CH_3$ | |
| 90 | 2-(4-Methoxyphenyl)-thiazolyl-4 | $-CH_2-$ | 1 | H | 4 | $OCH_3$ | 130–132 |
| 91 | 2-(4-Methoxyphenyl)-thiazolyl-4 | $-CH_2-$ | 1 | H | 4 | $CH_3$ | |

Fortsetzung

| Verbindung Nr. | Het | X | n | Z | Stellung der Harnstoffgruppe | R | Fp [°C] |
|---|---|---|---|---|---|---|---|
| 92 | 2-(3-Methylphenyl)-thiazolyl-4 | $-CH_2-$ | 1 | H | 3 | $OCH_3$ | 107–108 |
| 93 | 2-(3-Methylphenyl)-thiazolyl-4 | $-CH_2-$ | 1 | H | 3 | $CH_3$ | |
| 94 | 2-(3-Methylphenyl)-thiazolyl-4 | $-CH_2-$ | 1 | H | 4 | $OCH_3$ | 123–125 |
| 95 | 2-(3-Methylphenyl)-thiazolyl-4 | $-CH_2-$ | 1 | H | 4 | $CH_3$ | |
| 96 | 2-(2-Chlorphenyl)-thiazolyl-4 | $-CH_2-$ | 1 | H | 3 | $OCH_3$ | |
| 97 | 2-(2-Chlorphenyl)-thiazolyl-4 | $-CH_2-$ | 1 | H | 3 | $CH_3$ | |
| 98 | 2-(2-Chlorphenyl)-thiazolyl-4 | $-CH_2-$ | 1 | H | 4 | $OCH_3$ | 130–132 |
| 99 | 2-(2-Chlorphenyl)-thiazolyl-4 | $-CH_2-$ | 1 | H | 4 | $CH_3$ | |
| 100 | 3-(4-Nitrophenyl)-1,2,4-oxadiazolyl-5 | $-CH_2-$ | 1 | H | 4 | $OCH_3$ | 188–190 |
| 101 | 3-(4-Nitrophenyl)-1,2,4-oxadiazolyl-5 | $-CH_2-$ | 1 | H | 3 | $CH_3$ | |
| 102 | 3-(4-Chlorphenyl)-1,2,4-oxadiazolyl-5 | $-CH_2-$ | 1 | H | 3 | $OCH_3$ | |
| 103 | 3-(4-Chlorphenyl)-1,2,4-oxadiazolyl-5 | $-CH_2-$ | 1 | H | 3 | $CH_3$ | |
| 104 | 3-(4-Chlorphenyl)-1,2,4-oxadiazolyl-5 | $-CH_2-$ | 1 | H | 4 | $OCH_3$ | 140–142 |
| 105 | 3-(4-Chlorphenyl)-1,2,4-oxadiazolyl-5 | $-CH_2-$ | 1 | H | 4 | $CH_3$ | |
| 106 | 3-Methylisoxazolyl-5 | $-CH_2-$ | 1 | 2-Cl | 4 | $OC_2H_5$ | 77–79 |
| 107 | 3-Methylisoxazolyl-5 | $-CH_2-$ | 1 | 2-Cl | 4 | $C_2H_5$ | |
| 108 | 3-Methylisoxazolyl-5 | $-CH_2-$ | 1 | 2-Cl | 4 | $CH_2CH=CH_2$ | |

Fortsetzung

| Verbindung Nr. | Het | X | n | Z | Stellung der Harnstoffgruppe | R | Fp [°C] |
|---|---|---|---|---|---|---|---|
| 109 | 3-Methylisoxazolyl-5 | $-CH_2-$ | 1 | 2-Cl | 4 | $CH(CH_3)C{\equiv}CH$ | |
| 110 | 3-Methylisoxazolyl-5 | $-CH_2-$ | 1 | $2\text{-}CF_3$ | 4 | $OC_2H_5$ | |
| 111 | 2-(4-Chlorphenyl)-thiazol-4-yl | $-CH_2-$ | 1 | 2-Cl | 4 | $OCH_3$ | 160–161 |
| 112 | 2-(4-Chlorbenzyl)-thiazol-4-yl | $-CH_2-$ | 1 | 2-Cl | 4 | $OCH_3$ | 114–115 |
| 114 | 4-Methylthiazol-5-yl | $-CH_2CH_2-$ | 1 | H | 4 | $OCH_3$ | 120–122 |
| 115 | 4-Methylthiazol-5-yl | $-CH_2CH_2-$ | 1 | H | 4 | $CH_3$ | 111–112 |
| 116 | 4-Methylthiazol-5-yl | $-CH_2CH_2-$ | 1 | 2-Cl | 4 | $OCH_3$ | 128–130 |
| 117 | 4-Methylthiazol-5-yl | $-CH_2CH_2-$ | 1 | 2-Cl | 4 | $CH_3$ | 110–112 |
| 118 | 4-Methylthiazol-5-yl | $-CH_2CH_2-$ | 1 | $2\text{-}CF_3$ | 4 | $OCH_3$ | 87–89 |
| 119 | 4-Methylthiazol-5-yl | $-CH_2CH_2-$ | 1 | $2\text{-}CF_3$ | 4 | $CH_3$ | 90–93 |
| 120 | 1,2,4-Triazol-1-yl | $-CH_2CH_2-$ | 1 | H | 4 | $OCH_3$ | 117–118 |
| 121 | 1,2,4-Triazol-1-yl | $-CH_2CH_2-$ | 1 | H | 4 | $CH_3$ | 136–138 |
| 122 | 3-Methylisoxazolyl-5-yl | $-CH_2-$ | 1 | 2-Br | 4 | $OCH_3$ | 130–133 |
| 123 | 3-Methylisoxazolyl-5-yl | $-CH_2-$ | 1 | 3-Br | 4 | $OCH_3$ | 91–93 |
| 124 | 3-Methylisoxazolyl-5-yl | $-CH_2-$ | 1 | H | 3 | $OC_2H_5$ | 94–96 |
| 125 | 3-Phenyl-1,2,4-oxadiazol-5-yl | $-CH_2-$ | 1 | H | 4 | $OCH_3$ | 107–109 |
| 126 | 2-Methylthiazol-4-yl | $-CH_2-$ | 1 | 2-Cl | 4 | $OCH_3$ | 99–101 |

Fortsetzung

| Verbindung Nr. | Het | X | n | Z | Stellung der Harnstoffgruppe | R | Fp [°C] |
|---|---|---|---|---|---|---|---|
| 127 | 2-Methylthiazol-4-yl | $-CH_2-$ | 1 | 2-Br | 4 | $OCH_3$ | 102–104 |
| 128 | 2-Methylthiazol-4-yl | $-CH_2-$ | 1 | 2-$CF_3$ | 4 | $OCH_3$ | 119–122 |
| 129 | 5-Trifluormethylbenzthiazol-2-yl | $-CH_2CH_2-$ | 0 | H | 4 | $CH_3$ | |
| 130 | 5-Trifluormethylbenzthiazol-2-yl | $-CH_2CH_2-$ | 0 | H | 4 | $OCH_3$ | |
| 131 | 5-Trifluormethylbenzthiazol-2-yl | $-CH_2CH_2-$ | 0 | H | 3 | $CH_3$ | |
| 132 | 5-Trifluormethylbenzthiazol-2-yl | $-CH_2CH_2-$ | 0 | H | 3 | $OCH_3$ | |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 5 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 13 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteile Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 19 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 50 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 64 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus

einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 36 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 46 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 23 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder bei Nachauflaufanwendung erfolgen. Vorzugsweise werden die neuen Wirkstoffe nach dem Auflaufen oder während des Auflaufens der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,025 bis 10 kg/ha und mehr, vorzugsweise 0,1 bis 4,0 kg/ha, wobei sich die höheren Dosen besonders zur totalen Bekämpfung von Vegetationen eignen.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Die Aufwandmengen variieren je nach Verbindung; sie betragen ungefähr 3,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Für die Nachauflaufbehandlung zieht man die Testpflanzen je nach Wuchsform erst bis zu einer Wuchshöhe von 3 bis 15 cm an und behandelt sie danach. Die für die Nachauflaufanwendung eingesetzten Sojapflanzen zieht man im Gegensatz zu der übrigen Erde in einem mit Torfmull (peat) angereicherten Substrat an, um ein günstigeres Wachstum zu gewährleisten. Zur Nachauflaufbehandlung werden entweder direkt gesäte und in den gleichen Gefäßen aufgewachsene Pflanzen ausgewählt, oder aber sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff. Sie betragen 0,125, 0,25, 0,5 bzw. 1,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 30°C) und für solche gemäßigter Klimate 15 bis 25°C bevorzugt werden. Die Versuchsperiode erstreckt sich über 3 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Bei den Testpflanzen handelt es sich um Alopecurus myosuroides (Acker-Fuchsschwanzgras), Amaranthus retroflexus (Zurückgekrümmter Fuchsschwanz), Arachys hypogaea (Erdnüsse), Avena Fatua (Flughafer), Chenopodium album (Weißer Gänsefuß), Desmodium tortuosum, Echinochloa crus-galli (Hühnerhirse), Euphorbia geniculata (Südamerikan. Wolfsmilchart), Galium aparine (Klettenlabkraut), Glycine max. (Soja), Gossypium hirsutum (Baumwolle), Ipomoea spp. (Prunkwindearten), Lamium spp. (Taubnesselarten), Lolium multiflorum (Ital. Raygras), Mercurialis annua (einjähriges Bingelkraut), Nicandra physaloides (Giftbeere), Sesbania exaltata (Turibaum), Sinapis alba (Weißer Senf), Solanum nigrum (Schwarzer Nachtschatten), Triticum aestivum (Weizen), Setaria italica, Sida spinosa, Viola tricolor (Stiefmütterchen).

Bei Vorauflaufanwendung zeigen beispielsweise die Verbindungen Nr. 50, 56, 36, 52, 46, 21, 19, 5, 23, 64, 13 und 48 bei Aufwandmengen von 3,0 kg Wirkstoff/ha eine beachtliche herbizide Aktivität.

Bei Nachauflaufanwendung bekämpfen beispielsweise die Verbindungen Nr. 1, 9, 26 und 64 mit je 0,5 kg sowie die Verbindung Nr. 46 mit 1,0 kg/ha eine ganze Reihe von unerwünschten Pflanzen selektiv. Ebenso zeigen die Verbindungen Nr. 15, 48 und 50 mit 0,5 kg/ha, die Verbindungen Nr. 36 und 21

mit 0,25 kg/ha und die Verbindungen Nr. 19 und 23 mit 0,125 kg/ha bei Nachauflaufanwendung eine selektive herbizide Wirkung. Die Verbindung Nr. 52 zeigt bei Nachauflaufanwendung und niedriger Aufwandmenge eine sehr gute Wirkung gegen breitblättrige unerwünschte Pflanzen. Bei Aufwandmengen von 3,0 kg/ha zeigen die Verbindungen Nr. 7, 29, 38, 72, 75, 94, 106, 124, 125 eine gute herbizide Wirkung.

In Anbetracht der Verträglichkeit und der Vielseitigkeit der Applikationsmethoden, können die Verbindungen der Formel I bzw. sie enthaltende herbizide Mittel noch in einer weiteren Zahl von Kulturpflanzen zur Beseitigung unerwünschter Pflanzen eingesetzt werden.

In Betracht kommen beispielsweise folgende Kulturen:

| Botanischer Name | Deutscher Name |
|---|---|
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Camellia sinensis | Teestrauch |
| Carthamus tinctorius | Saflor – Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |

Fortsetzung

| Botanischer Name | Deutscher Name |
|---|---|
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstunsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

## Patentansprüche

1. Harnstoffderivate der Formel

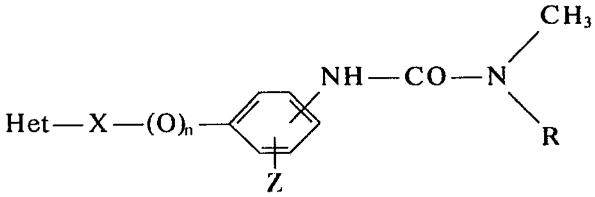

(I)

in der

R Wasserstoff, einen Alkyl- oder Alkoxyrest mit bis zu 4 Kohlenstoffatomen, Allyl oder 1-Methylprop-2-inyl,

X einen unverzweigten oder verzweigten Alkylenrest mit bis zu 4 Kohlenstoffatomen,

Z Wasserstoff, Halogen, Methyl, Trifluormethyl,

Het einen gegebenenfalls durch Halogen, Alkyl, Halogenalkyl, Alkoxy, Halogenalkoxy, Alkoxyalkyl oder Alkoxycarbonyl mit jeweils bis zu 5 Kohlenstoffatomen oder durch gegebenenfalls substituiertes Phenyl oder durch gegebenenfalls substituiertes Benzyl substituierten Isoxazol-, Oxazol-, Thiazol-, Oxadiazol-, Thiadiazol-, Pyrazol- oder Triazolring oder einen entsprechenden benzoanellierten, gegebenenfalls in gleicher Weise substituierten Fünfringheterocyclus und
n 0 oder 1 bedeuten.

2. Harnstoffderivate der Formel I gemäß Anspruch 1, dadurch gekennzeichnet, daß Het für einen gegebenenfalls durch Halogen, Alkyl, Halogenalkyl, Alkoxy, Halogenalkoxy, Alkoxyalkyl oder Alkoxycarbonyl mit jeweils bis zu 5 Kohlenstoffatomen oder durch gegebenenfalls substituiertes Phenyl oder durch gegebenenfalls substituiertes Benzyl substituierten Benzthiazolylrest steht.

3. Verfahren zur Herstellung von Harnstoffderivaten der Formel I gemäß Anspruch 1, in der n 1 bedeutet, dadurch gekennzeichnet, daß man eine Verbindung der Formel

Het—X—Hal (II)

in der Het und X die im Anspruch 1 genannten Bedeutungen haben und Hal für Halogen steht, mit einem Phenol der Formel

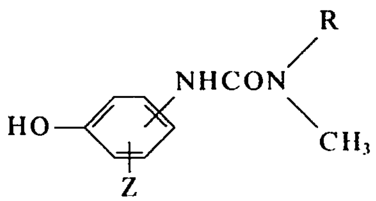
(III)

in der R und Z die im Anspruch 1 genannten Bedeutungen haben,
in einem inerten Lösungsmittel bei einer Temperatur zwischen 0 und 150°C umsetzt.

4. Verfahren zur Herstellung von Harnstoffderivaten der Formel I gemäß Anspruch 1, in der n 1 bedeutet, dadurch gekennzeichnet, daß man Verbindungen der Formel

Het—X—OH (IV)

in der Het und X die im Anspruch 1 genannten Bedeutungen haben,
mit Halogennitroaromaten der Formel

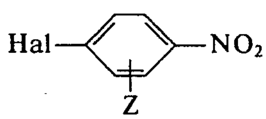
(V)

in der Z die oben genannten Bedeutungen hat und Hal für Halogen steht, bei einer Temperatur zwischen 0 und 150°C umsetzt, die Nitroverbindungen reduziert und die so erhaltenen Anilinderivate mit einem Isocyanat der Formel

RNCO

oder einem Carbaminsäurechlorid der Formel

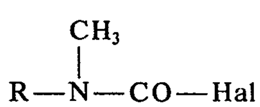

wobei R jeweils die im Anspruch 1 genannten Bedeutungen hat und Hal für Halogen steht, umsetzt.

5. Verfahren zur Herstellung von Harnstoffderivaten der Formel I gemäß Anspruch 1, in der n 0 bedeutet, dadurch gekennzeichnet, daß man einen Aldehyd der Formel

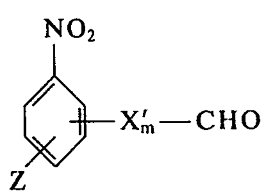
(VIII)

in der X' einen unverzweigten oder verzweigten Alkylenrest mit 1 oder 2 Kohlenstoffatomen und m 0 oder 1 bedeuten und Z die im Anspruch 1 genannten Bedeutungen, hat mit einer aktiven Methylenverbindung der Formel

$$Het—CH_3 \qquad (IX)$$

in der Het die im Anspruch 1 genannten Bedeutungen hat, umsetzt, das Reaktionsprodukt hydriert und durch Umsetzung mit einem Carbaminsäurehalogenid der Formel

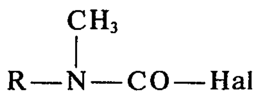

oder einem Isocyanat der Formel

RNCO

wobei R jeweils die im Anspruch 1 genannten Bedeutungen hat und Hal für Halogen steht, in das Harnstoffderivat überführt.

6. Herbizid, enthaltend ein Harnstoffderivat der Formel I gemäß Anspruch 1.

7. Herbizides Mittel, enthaltend inerte Zusatzstoffe und 0,1 bis 95 Gew.-% eines Harnstoffderivates der Formel

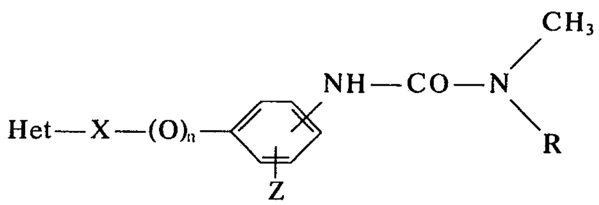

(I)

in der

R Wasserstoff, einen Alkyl- oder Alkoxyrest mit bis zu 4 Kohlenstoffatomen, Allyl, 1-Methyl-prop-2-inyl,
X einen unverzweigten oder verzweigten Alkylenrest mit bis zu 4 Kohlenstoffatomen,
Z Wasserstoff, Halogen, Methyl, Trifluormethyl,
Het einen gegebenenfalls durch Halogen, Alkyl, Halogenalkyl, Alkoxy, Halogenalkoxy, Alkoxyalkyl oder Alkoxycarbonyl mit jeweils bis zu 5 Kohlenstoffatomen oder durch gegebenenfalls substituiertes Benzyl substituierten Isoxazol-, Oxazol-, Thiazol-, Oxadiazol-, Thiadiazol-, Pyrazol- oder Triazolring oder einen entsprechenden benzoanellierten, gegebenenfalls in gleicher Weise substituierten Fünfringheterocyclus und
n o Oder 1 bedeuten.

8. Herbizides Mittel nach Anspruch 7, dadurch gekennzeichnet, daß es ein Harnstoffderivat der Formel I enthält, wobei Het für einen gegebenenfalls durch Halogen, Alkyl, Halogenalkyl, Alkoxy, Halogenalkyl, Alkoxyalkyl oder Alkoxycarbonyl mit jeweils bis zu 5 Kohlenstoffatomen der durch gegebenenfalls substituiertes Phenyl oder durch gegebenenfalls substituiertes Benzyl substituierten Benzthiazolylrest steht.

9. Verfahren zur Bekämpfung unerwünschten Pflanzenwuchses, dadurch gekennzeichnet, daß man die unerwünschten Pflanzen oder die von unerwünschtem Pflanzenwuchs freizuhaltende Fläche mit einer herbizid wirksamen Menge eines Harnstoffderivats der Formel I gemäß Anspruch 1 behandelt.

**Claims**

1. A urea derivative of the formula

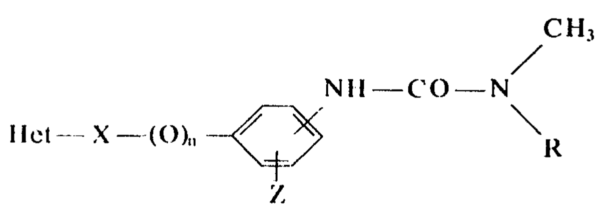

(I)

where

R is hydrogen, or alkyl or alkoxy of up to 4 carbon atoms, allyl or 1-methyl-prop-2-ynyl,
X is straight-chain or branched alkylene of up to 4 carbon atoms,
Z is hydrogen, halogen, methyl or trifluoromethyl and
Het is an isoxazole, oxazole, thiazole, oxadiazole, thiadiazole, pyrazole or triazole ring optionally substituted by halogen, or alkyl, haloalkyl, alkoxy, haloalkoxy, alkoxyalkyl or alkoxycarbonyl, each of up to 5 carbon atoms, or by optionally substituted phenyl or by optionally substituted benzyl, or is a corresponding benzyl-fused 5-membered heterocyclic radical, which may be substituted in the same way, and
n is 0 or 1.

2. A urea derivative of the formula I as claimed in claim 1, where Het is benzthiazolyl which is unsubstituted or substituted by halogen, alkyl, haloalkyl, alkoxy, haloalkyl, alkoxyalkyl or alkoxycarbonyl, each of up to 5 carbon atoms, or by optionally substituted phenyl, or by optionally substituted benzyl.

3. A process for the preparation of a urea derivative of the formula I as claimed in claim 1, where n is 1, wherein a compound of the formula

Het—X—Hal (II)

where Het and X have the meanings given in claim 1, and Hal is halogen, is reacted with a phenol of the formula

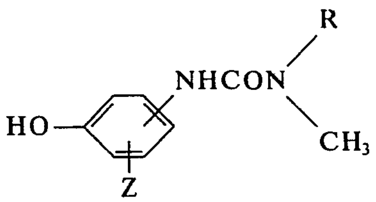

(III)

where R and Z have the meanings given in claim 1, in an inert solvent at from 0 to 150°C.

4. A process for the preparation of a urea derivative of the formula I as claimed in claim 1, where n is 1, wherein a compound of the formula

Het—X—OH (IV)

where Het and X have the meanings given in claim 1, is reacted at from 0 to 150°C with a halonitroaromatic of the formula

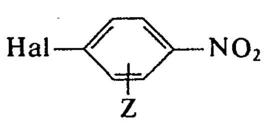

(V)

where Z has the above meanings and Hal is halogen, the nitro compound is reduced and the resulting aniline derivative is reacted with an isocyanate of the formula

RNCO

or with a carbamyl halide of the formula

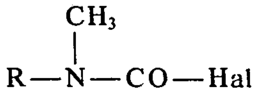

where R in each formula has the meanings given in claim 1, and Hal is halogen.

5. A process for the preparation of a urea derivative of the formula I as claimed in claim 1, where n is 0, wherein an aldehyde of the formula

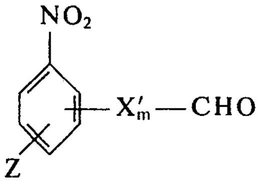

(VIII)

where X′ is straight-chain or branched alkylene of 1 or 2 carbon atoms, m is 0 or 1 and Z has the meanings given in claim 1, is reacted with an active methylene compound of the formula

$$Het—CH_3 \quad (IX)$$

where Het has the meanings given in claim 1, the reaction product is hydrogenated and converted into the urea derivative by reaction with a carbamyl halide of the formula

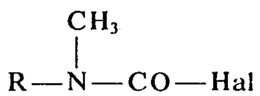

or with an isocyanate of the formula

RNCO

where R in each formula has the meanings given in claim 1, and Hal is halogen.

6. A herbicide containing a urea derivative of the formula I as claimed in claim 1.

7. A herbicide containing inert additives and from 0.1 to 95 % by weight of a urea derivative of the formula

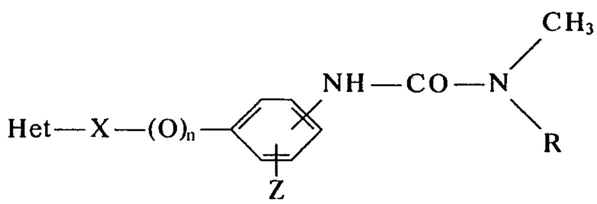

(I)

where

R is hydrogen, or alkyl or alkoxy of up to 4 carbon atoms, allyl or 1-methyl-prop-2-ynyl,
X is straight-chain or branched alkylene of up to 4 carbon atoms,
Z is hydrogen, halogen, methyl or trifluoromethyl and
Het is an isoxazole, oxazole, thiazole, oxadiazole, thiadiazole, pyrazole or triazole ring optionally substituted by halogen, or alkyl, haloalkyl, alkoxy, haloalkoxy, alkoxyalkyl or alkoxycarbonyl, each of up to 5 carbon atoms, or by optionally substituted phenyl or by optionally substituted benzyl, or is a corresponding benzyl-fused 5-membered heterocyclic radical, which may be substituted in the same way, and
n is 0 or 1.

8. A herbicidal agent as claimed in claim 7, containing a urea derivative of the formula I where Het is benzthiazolyl which is unsubstituted or substituted by halogen, alkyl, haloalkyl, alkoxy, haloalkoxy, alkoxyalkyl or alkoxycarbonyl, each up to 5 carbon atoms, or by optionally substituted phenyl, or by optionally benzyl.

9. A process for combating unwanted plant growth, wherein the unwanted plants or the area to be kept free from unwanted plant growth are treated with a herbicidally effective amount of a urea derivative of the formula I as claimed in claim 1.

## Revendications

1. Dérivés d'urée de formule

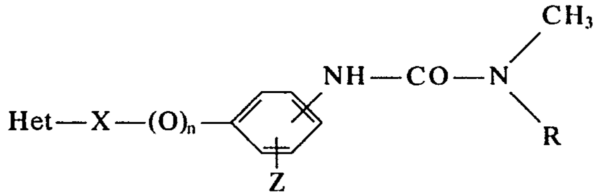

(I)

dans laquelle

R représente hydrogène, un reste alkyle ou alcoxy ayant jusqu'à 4 atomes de carbone, allyle ou 1-méthyl-prop-2-inyle,
X un reste alkylène, non ramifié, ayant jusqu'à 4 atomes de carbone,
Z hydrogène, halogène, méthyle, trifluorométhyle,
Het un noyau isoxazole, oxazole, thiazole, oxadiazole, thiadiazole, pyrazole ou triazole, éventuellement substitué par halogène, alkyle, halogénoalkyle, alcoxy, halogénoalcoxy, alcoxyalkyle ou alcoxycarbonyle ayant jusqu'à 5 atomes de carbone ou par phényle, éventuellement substitué, ou par benzyle, éventuellement substitué, ou un hétérocycle à cinq noyaux, correspondant, benzocondensé, éventuellement substitué de la même manière, et
n représente 0 ou 1.

2. Dérivés d'urée de formule I selon la revendication 1, caractérisés par le fait que Het est mis pour un reste benzothiazolyle, éventuellement substitué par halogène, alkyle, halogénoalkyle, alcoxy, halogénoalcoxy, alcoxyalkyle ou alcoxycarbonyle ayant jusqu'à 5 atomes de carbone ou par phényle éventuellement substitué ou par benzyle éventuellement substitué.

3. Procédé de préparation de dérivés d'urée de formule I selon la revendication 1, dans laquelle n représente 1, caractérisé par le fait que l'on fait réagir un composé de formule

$$\text{Het—X—Hal} \qquad \text{(II)}$$

dans laquelle Het et X ont les significations indiquées dans la revendication 1 et Hal représente halogène, avec un phénol de formule

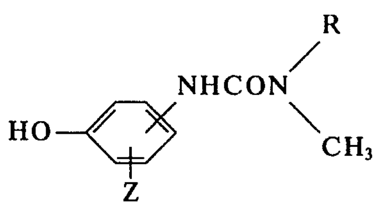

(III)

dans laquelle R et Z ont les significations indiquées dans la revendication 1, dans un solvant inerte, à une température comprise entre 0 et 150°C.

4. Procédé de préparation de dérivés d'urée de formule I selon la revendication 1, dans laquelle n représente 1, caractérisé par le fait que l'on fait réagir des composés de formule

$$\text{Het—X—OH} \qquad \text{(IV)}$$

dans laquelle Het et X ont les significations indiquées dans la revendication 1 avec des halogénonitroaromates de formule

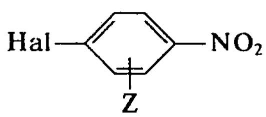

(V)

dans laquelle Z a les significations indiquées plus haut et Hal représente halogène, à une température comprise entre 0 et 150°C, on réduit les composés nitrés et on fait réagir les dérivés d'aniline ainsi obtenus avec un isocyanate de formule

RNCO

ou un halogénure d'acide carbamique de formule

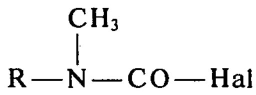

R ayant les significations indiquées dans la revendication 1 et Hal représentant halogène.

5. Procédé de préparation de dérivés d'urée de formule I selon la revendication 1, dans lequel n représente 0, caractérisé par le fait que l'on fait réagir un aldéhyde de formule

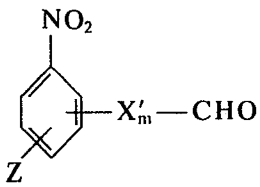

(VIII)

dans laquelle X' représente un reste alkylène, ramifié ou non ramifié, à 1 à 2 atomes de carbone et m représente 0 ou 1 et Z a les significations indiquées dans la revendication 1, avec un composé méthylénique actif de formule

$$\text{Het—CH}_3 \qquad \text{(IX)}$$

dans laquelle Het a les significations indiquées dans la revendication 1, on hydrogène le produit de réaction et, par réaction avec un halogénure d'acide carbamique de formule

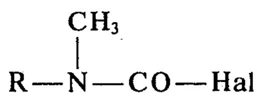

ou un isocyanate de formule

RNCO

R ayant les significations indiquées dans la revendication 1 et Hal représentant halogène, on le transforme en dérivé d'urée.

6. Herbicide, contenant un dérivé d'urée de formule I selon la revendication 1.

7. Agent herbicide, contenant des additifs inertes et 0,1 à 95 % en poids d'un dérivé d'urée de formule

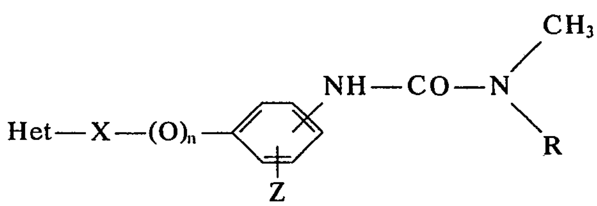

(I)

dans laquelle

R représente hydrogène, un reste alkyle ou alcoxy, ayant jusqu'à 4 atomes de carbone, allyle ou 1-méthyl-prop-2-inyle,

X un reste alkylène, non ramifié ou ramifié, ayant jusqu'à 4 atomes de carbone,

Z hydrogène, halogène, méthyle, trifluorométhyle,

Het un noyau isoxazole, oxazole, thiazole, oxadiazole, thiadiazole, pyrazole ou triazole, éventuellement substitué par halogène, alkyle, halogénoalkyle, alcoxy, halogénoalcoxy, alcoxyalkyle ou alcoxycarbonyle ayant jusqu'à 5 atomes de carbone ou par phényle, éventuellement substitué, ou par benzyle, éventuellement substitué, ou un hétérocycle à cinq noyaux, correspondant, benzocondensé, éventuellement substitué de la même manière, et

n représente 0 ou 1.

8. Agent herbicide selon la revendication 7, caractérisé par le fait qu'il contient un dérivé d'urée de formule I, où Het est mis pour un reste benzothiazolyle éventuellement substitué par halogène, alkyle, halogénoalkyle, alcoxy, halogénoalcoxy, alcoxyalkyle ou alcoxycarbonyle ayant jusqu'à 5 atomes de carbone ou par phényle éventuellement substitué ou par benzyle éventuellement substitué.

9. Procédé pour combattre le développement de plantes indésirables, caractérisé en ce que l'on traite

les plantes indésirables ou la surface, sur laquelle le développement des plantes indésirables doit être empêché, avec une quantité efficace du point de vue herbicide d'un dérivé d'urée de la formule I selon la revendication 1.